(19) Europäisches Patentamt
European Patent Office
Office européen des brevets

(11) **EP 2 110 076 A1**

(12) **EUROPEAN PATENT APPLICATION**

(43) Date of publication:
**21.10.2009 Bulletin 2009/43**

(51) Int Cl.:
*A61B 5/05* [(2006.01)]       *A61B 8/00* [(2006.01)]
*G01N 21/17* [(2006.01)]

(21) Application number: **08006528.7**

(22) Date of filing: **31.03.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **19.02.2008 US 66187 P**

(71) Applicant: **Helmholtz Zentrum München Deutsches Forschungszentrum für Gesundheit und Umwelt (GmbH)**
**85764 Neuherberg (DE)**

(72) Inventors:
• **Razansky, Daniel**
  **80335 München (DE)**
• **Ntziachristos, Vasilis**
  **81679 München (DE)**

(74) Representative: **Hertz, Oliver**
  **v. Bezold & Partner**
  **Patentanwälte**
  **Akademiestrasse 7**
  **80799 München (DE)**

(54) **Method and device for near-field dual-wave modality imaging**

(57)    A method of near-field imaging a region of interest in an object comprises the steps emitting an input of near-field electromagnetic energy from a near-field source device into the region of interest, detecting a mechanical wave response generated in the region of interest in response to the near-field electromagnetic energy input, and providing image data representing an image of the region of interest on the basis of the mechanical wave response. Furthermore, a device for near-field imaging the region of interest is described. The invention relies on near-field exposure of the object to electromagnetic sources of safe energies. The physical basis of the image formation is the thermoacoustic phenomenon, which utilizes radio-frequency (RF) energy deposition onto the object under investigation. Subsequently, the absorbed electromagnetic energy generates acoustic response, which can be detected and used for forming volumetric visualization (image) of the underlining tissue absorption properties.

FIG. 1

**Description**

**Field of the invention**

The present invention relates to a method and a device for near-field imaging of a region of interest using near-field electromagnetic waves. The method, termed Near-field Radiofrequency Tomography (NRT), and the device are capable of producing tomographic images of e.g. anatomical, functional and molecular characteristics of biological objects, like biological tissues, or non-biological objects.

**Background of the invention**

Medical imaging has been used for more than a century to non-invasively assess tissue pathology. The progress of many diseases is associated with conformational, physiological and molecular changes in the surrounding environment that sustain contrast to a wide energy spectrum. Originally, medical imaging targeted structure, wherein X-ray imaging and computerized tomography (CT), magnetic resonance imaging (MRI) and ultrasonography have traditionally looked into architectural tissue malformations to identify disease. Interest however was later steered towards imaging physiology as a complement or surrogate to structural signatures. Targeting vascularization, tissue permeability, blood flow and other functional parameters revealed information on disease function and enhanced the diagnostic information available. More recently keen interest has developed for imaging of pathogenesis in intact tissues at the molecular level. Imaging of cellular and sub-cellular function is increasingly becoming a key technology in preclinical imaging and drug discovery and holds strong potential for large-scale clinical propagation as well.

There is a wealth of medical imaging technologies, each offering different characteristics and application indications. Broad classification can be based on the use of ionizing (high-energy) radiation such X-ray Computed Tomography (XCT), Positron Emission Tomography (PET) and Single Photon Emission Tomography (SPECT) vs. modalities utilizing non-ionizing radiation, such as Magnetic Resonance Imaging (MRI), optical imaging tomography and ultrasound (US).

There are several efforts to turn the existing imaging modalities into molecularly-sensitive imaging methods suitable for whole-body clinical imaging. Molecular probes have been developed for PET and SPECT methods, operating as stand-alone or in hybrid implementations with CT methods. The major disadvantage of those methods is the ionizing radiation they employ and the need for cyclotron facilities, advanced radio-chemistry expertise and the radio-decay of the probes used, which is inappropriate for storing agents or for longitudinal monitoring studies. Similarly there is significant progress in developing molecular-sensing MRI using probes that can sense molecular moieties. Generally, MRI probes need cannot be detected with high sensitivity, which compromises the range of applications of this approach. Optical techniques are also considered for molecular sensing but they generally suffer for low penetration depth in tissues and limited resolution when imaging deep in tissue, which may limit clinical applications to tissue surface imaging and invasive or endoscopic applications.

Newer imaging techniques such as Thermoacoustic imaging (TAI) and Photoacoustic Imaging (PAI) also fall under the "non-ionizing" imaging techniques. In its classical form, thermoacoustic imaging relies on the thermoacoustic phenomenon, which was discovered by A. G. Bell in 1881, in particular on the generation of broadband acoustic radiation by instantaneous expansion of tissue, after absorption of a short pulse of electromagnetic energy. The magnitude of the induced acoustic waves is proportional to local energy deposition and thermoelastic properties of matter. The acoustic wave frequency is mainly dependent upon the spatial frequency of optical absorption variations and duration of the pulse. The term thermoacoustic tomography (TAT) is frequently related to radio-frequency and microwaves irradiating spectra, whereas photoacoustic tomography (PAT) is usually used whenever the object is irradiated with pulsed lasers in the visible or near-infrared bands.

A. Rosencwaig (U.S. 4,255,971) was among the first to describe an imaging application based on the thermoacoustic phenomenon, operating using electromagnetic energy radiation. Several subsequent implementations of thermoacoustic imaging mainly utilized far-field radiation by employing short narrowband pulses of microwave radiation in the range from 400 MHz to 3 GHz. For example, U.S. 6,567,688 discloses a thermoacoustic CT scanner that transmits sub-microsecond pulses of microwave radiation at 434 MHz into the tissue. The resulting thermoacoustic response is detected by multiple acoustic transducers arranged on a rotatable imaging bowl. The detected signals at multiple locations around the imaged object are then combined to form an image of tissue absorption. A similar approach is suggested in U.S. 6,216,025 except that pulses with carrier frequency of 3 GHz were used. Also, the image is acquired by translating the imaged sample in front of an ultrasonic transducer that detects the induced thermoacoustic response.

It was recently shown that both PAT and TAT have the potential to image animal or human tissues with both high contrast and high spatial resolution unlike other pure electromagnetic/optical or pure ultrasonic modalities. In particular TAT has shown great potential in high-contrast imaging of deep tissues, thus overcoming limitations of pure optical/electromagnetic or pure ultrasonic methods. RF and microwave spectra offer good contrast in biological samples with e.g. one order of magnitude difference of thermoacoustic response between fat and muscle tissues. Another example is cancerous breast tissue which is found to be more than twice as absorbing as the normal surrounding breast. The electromagnetic energy in the RF and microwave part of the spectrum penetrates well the human body so that thermoacoustic imaging is suitable for whole-body clinical applications.

Despite some promising characteristics of TAT, it has not become a mainstream imaging approach due to several shortcomings. First, operation at the far-field imposes significant losses due to the reflection of the radiated energy. Since a significant part of the energy is lost on its way to the object, thermoacoustic signal generation becomes inefficient, which significantly compromises the signal-to-noise ratio at practical and safe power levels. Second, existing thermoacoustic tomography methods utilize irradiation of the object by using narrow-band microwave radiation, i.e. electromagnetic energy deposition in the far-field. One of the important aspects in thermoacoustic tomography is the duration of the electromagnetic pulse, which has to be as short as possible in order to increase the spatial resolution of the method. Conversely, significantly reducing the pulse duration, to achieve high spatial resolution leads to small or undetectable thermoacoustic signal levels. Typically, with carrier frequency in the microwave band, peak power of several Megawatts is required in order to have good signal-to-noise ratio (SNR) and spatial resolution on the order of 100 microns. This, in turn, requires dedicated and extremely expensive pulse generation and amplification equipment and may also impose safety concerns in regard to the instrumentation used. For this reason it is more common to relax the spatial resolution requirements to several millimeters by allowing longer pulses and reducing peak powers to the order of tens of Kilowatts. Overall the low SNR, usually obtained in the existing methods, hinders their practical implementation in the field.

On the different end of the penetration spectrum of imaging methods, near-field imaging usually refers to different sub-wavelength microscopy methods capable of going beyond diffraction-limited resolution. One of the techniques is based on the concept of forcing a wave through an aperture smaller than a wavelength of the radiation used. This concept is applied with scanning tunneling microscopy (e. g. U.S. 4 343 993) and scanning near-field optical microscopy (SNOM). A slightly different approach utilizes attenuated total reflection or surface plasmon resonance interactions in noble metal nano-films in order to sense or form two-dimensional images of structures as small as one-hundredth of the wavelength employed. For example, U.S. 7,005,653 discloses a method and apparatus for near-field intra-cellular apertureless tomographic imaging, which uses sub-wavelength nano-particle in a cell, which generates tomographic projections. A detector detects and collects high-frequency details from evanescent field interactions of the nano-particle with surrounding molecules and provides near-field imagery of a cell volume.

Near-field imaging is far from being limited to the optical spectrum. In fact, the first sub wavelength aperture scanning microscope operating in the microwave region of the electromagnetic spectrum was demonstrated in 1972 by L. A. Ash and G. Nicholls from the University College in London (Ash, E.A., et al., "Super-Resolution Aperture Scanning Microscope", Nature, 237, pp. 510-512, June 1972). Utilizing microwaves, with a wavelength of 3 cm, passing through a probe-forming aperture of 1.5 mm, the probe was scanned over a metal grating having periodic line features. Both the 0.5-mm lines and 0.5-mm gaps in the grating were easily resolvable, demonstrating sub-wavelength resolution having approximately one-sixtieth (0.017) the period of the imaging wavelength. The same principles apply to near-field imaging using fields of non-electromagnetic nature. For instance, in scanning acoustic microscopy, an acoustic wave is produced in a tiny area just in the vicinity of surface (near-field area) through different interaction mechanisms. By detecting this acoustic wave, one can gain the acoustic properties of materials at a high resolution, not diffraction-limited by the wavelength used.

All the above near-field imaging methods are mainly intended for surface limited microscopic studies. Macroscopic tomographic methods, capable of large-scale volumetric visualization, do not normally operate in the near-field regime. One of the exceptions is magnetic resonance imaging. This approach can also be considered as near-field imaging modality since the object under investigation is placed in the near-field of RF coils, although the underlining imaging principles are fundamentally different than those of the other near-field detection methods, due to image formation based on weak tissue magnetization utilizing strong magnetic field. Diffuse Optical Tomography methods, including Fluorescence-mediated Molecular Tomography (FMT), can also be considered as near-field tomography methods, since they image in the sub-wavelength region of the diffusion-wave frequency employed and place the object of interest within the near-field of intensity modulated laser sources or the equivalent fields generated by short photon pulses in the picosecond and sub-picosecond range.


**Objective of the invention**


The objective of the invention is to provide new technology and methodology that fundamentally improve the shortcomings of established imaging techniques and offering an imaging approach that can find wide utility as an imaging method of tissues or other imaging objects.

This objective is reached with Near-field Radiofrequency Tomography (NRT), in particular an imaging method and an imaging device comprising the features of the independent claims. Advantageous embodiments of the invention are defined in the dependent claims.

**Summary of the invention**

According to a first aspect, the invention is based on the general technical teaching of providing a method of near-field imaging of a region of interest (ROI) in an object, whereas the ROI is subjected to an input of near-field electromagnetic energy, a mechanical wave response is detected in response to the near-field electromagnetic energy input in the ROI, and signals representing the detected mechanical wave response are converted into image data representing an image of the ROI. The invention relies on near-field exposure of the object or tissue to electromagnetic sources of safe energies and allows image formation based on the mathematical treatment of detected data generated as readings of pressure waves generated by near-field energy absorption from tissue elements. The physical basis of Near-field Radiofrequency Tomography (NRT) is the ability to efficiently couple energy in the region of interest because of utilization of technology that operates in the near-field, i.e. in a non-radiative area. NRT practical implementations utilize appropriate technology to produce ultra-short electromagnetic pulses, with very favorable and practical characteristics. These pulse characteristics allow operation at frequencies of optimal energy coupling to tissue and to agents that generate high structural, functional or molecular contrast. Operation of these pulses in the near-field offers improved coupling to tissue, even through air (i.e. in the absence of water as an energy delivering medium), and utilizing safe, practical, portable and cost-effective implementations that allow for high dissemination of the technology in research, clinical or other investigational areas. NRT makes significantly more adept use of the thermoacoustic phenomenon, compared to conventional thermoacoustic or photoacoustic imaging methods, offering a method of significantly higher SNR and resolution. Subsequently, the absorbed electromagnetic energy generates acoustic response, which can be detected and used for forming volumetric visualization (image) of the underlining tissue absorption properties. Near-field RF energy can easily penetrate tissues, such as the human body, making the method suitable for clinical imaging applications. In addition, the particular technology considered herein can efficiently select imaging settings and target resolution so that it can be adapted to human as well as to animal imaging.

According to a second aspect of the invention, an imaging device is provided, which in particular is adapted for conducting the near-field imaging method according to the above first aspect and which comprises a near-field source device creating and emitting a near-field electromagnetic energy to be input into a ROI, a detector device, which is arranged for detecting a mechanical wave response in the ROI, and an image processing device converting mechanical wave response signals into image data representing an image of the ROI. The near-field source device generally comprises at least one emitting element being adapted for creating and emitting electromagnetic energy into the surrounding or contained medium. Preferably, the near-field source device includes at least one antenna element, at least one phased array, at least one resonant cavity, and/or at least one waveguide.

Compared to other near-field techniques, inventive NRT allows penetration deep in tissues and makes the technique suitable for animal and human imaging, with many potential biological and medical applications, although the methodology can be used outside the biomedical field as well, in particular in all media that allow electromagnetic and acoustic wave propagation, e. g. in a work piece, a consumer good or a geological structure.

Compared to MRI, also an assumed near-field method, inventive NRT offers a significantly simpler and cost-effective technology of similar or better resolution performance and superior sensitivity. Compared to diffuse optical tomography methods, including Fluorescence-mediated Molecular Tomography, the near-field method of the invention offers significant improvement in resolution and overall penetration depth that can be achieved. Importantly, the present invention can be used as a clinical scanner implementation, as a non-invasive portable device or as an endoscopic, laparoscopic or other invasive device for tissue imaging.

The present invention drastically improves the utility of the thermoacoustic phenomenon for tissue imaging by placing the imaged object in the immediate vicinity (near-field) of the electromagnetic source, thus, allowing for improved coupling of the energy into the object, thus increasing the SNR available and improving detection sensitivity performance. Compared to thermoacoustic tomography, which also makes use of the thermoacoustic phenomenon, NRT overcomes many of the limitations of the method for practical, highly efficient application. Advantageously, far-field reflections are completely avoided, which occur with the conventional techniques. As an impedance mismatch can be ignored contrary to the conventional techniques, near-field electromagnetic energy can be coupled into the object with an increased efficiency of up to 60 % or more.

Of particular importance is the use of extrinsic energy absorbing agents or probes, which can be introduced into the region of interest imaged to improve the contrast or to demarcate a particular function, cellular, sub-cellular or molecular process. These agents can be contrast agents or modified contrast agents that have a targeted capacity, a preferential accumulation or bio-distribution capacity or modify their ability to impart contrast in response to the presence or absence of a biomarker or a tissue moiety of interest. These agents can be administered locally or systemically.

Compared to other medical modalities, NRT utilizes fundamentally different energies and image formation methods, while offering highly competitive or superior features. Compared to ultrasound for example it yields the same level of spatial resolution, but with significantly higher sensitivity, contrast and ability to resolve molecular activity via the use of nanoparticles. The sensitivity is improved as the ultrasound travels one way only through the object, so that any amplitude lost is reduced compared with conventional ultrasound imaging. Compared to nuclear imaging methods, the technique operates at the absence of ionizing radiation with significantly higher resolution.

Contrary to previous implementations of near-field imaging with measuring the same type of energy and spectrum that was used for the excitation of the sample, the inventive imaging method and device utilize near-field electromagnetic waves as a source and corresponding mechanical waves for detection. Therefore, the imaging method is a near-field dual wave modality imaging, whereas it is the electromagnetic part of the inventive system which operates in the near-field, whereas the mechanical wave / acoustic field can preferably operate, but not limited, to the far field. For simplicity, the method is termed herein Near-field Radiofrequency Tomography (NRT), a name that encompasses all aspects of the invention taught herein. The term NRT is used even if frequencies or wavelengths beyond "radio-frequency" are used.

Unless otherwise defined, all technical terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

According to a preferred embodiment of the invention, the near-field electromagnetic energy is created as a pulse-shaped radio-frequency signal. Advantageously, pulse-shaped signals allow an adjustment of duration of the energy input and accordingly an adjustment of the spatial resolution. In the preferred embodiment of this invention, when applied to imaging local biological tissue or animals, a radio-frequency signal is created, which comprises electromagnetic impulses having a duration in the range of 10 ps to 10 $\mu$s, in particular in the range of 100 ps to 1 $\mu$s, like e.g. 500 ps to 50 ns. As an advantage, electromagnetic impulses may provide a spatial resolution towards 100 $\mu$m or even down to 30 $\mu$m, 10 $\mu$m or below depending on the pulse width utilized and imaging depth. As a further advantage, electromagnetic impulses can be provided as broadband signals having high frequency (HF), very high frequency (VHF) or ultra high frequency (UHF) content in the frequency band between 3 MHz to 3 GHz, in particular VHF content from 30 MHz to 300 MHz. The broadband excitation of the ROI has a particular advantage in terms of creating thermoacoustic contrast with an extended range of media in the ROI. Second, frequency content in the VHF band will be able to penetrate for several tens of centimeters deep into biological tissue, thus making it possible to create high resolution and contrast near-field absorption images e. g. of deeply seated animal and human organs and structures, functions or cellular and molecular events. Furthermore, the short duration impulses can be generated with a generator device having an essentially simplified and low-cost structure compared with conventional techniques. As an example, a triggered spark gap coupled with the near-field source device can be used as the generator device, as also taught herein. Finally, utilization of the VHF band opens a possibility of non-contact imaging of objects without using a matching fluid or other medium. This is due to the fact that coupling electromagnetic radiation to an absorbing object inside an air medium is much easier for VHF as opposed to microwaves where most of the radiated power will be reflected from the surface.

According to an alternative NRT embodiment, the pulse-shaped radio-frequency signal comprises burst signals (pulses) with duration in the range of 1 ns to 10 $\mu$s. In this case, a spatial resolution of about 1-2 mm or down to 100 $\mu$m can be obtained with essentially reduced energy compared with the conventional techniques.

Preferably, at least one signal parameter of the pulse-modulated radio-frequency signal can be modulated before the input into the ROI. In particular, the signal parameter may comprise at least one of the amplitude, frequency, phase and digital modulation of the radio-frequency signal. Advantageously, modulating the signal parameter allows a facilitated adjustment of imaging conditions, in particular with regard to signal-to-noise ratio, penetration depth, spatial resolution and image contrast.

According to another alternative NRT embodiment, the near-field energy is introduced into the region of interest by means of a modulated intensity source. In this case, spatial information regarding near-field absorption can be extracted by performing at least one of the amplitude, frequency, phase or digital modulation of the radio-frequency signal and subsequently detecting the modulated mechanical response.

The above pulse duration and other frequency parameters of the electromagnetic energy source are given as an example only. They will be adjusted depending on actual dimensions of the imaged object or region of interest, type of imaging device and antenna and coupling medium. While the VHF frequency band excitation is mostly suited for small animal and partial human body imaging, whole-body imaging of humans and larger animals will require lower frequency content for better penetration and uniformity of excitation. In other words, the preferred frequency content of the excitation energy is inversely proportional to the size of the imaged object or region.

According to a further advantageous NRT embodiment, an environment of the object to be investigated, including the near-field source device and the ROI is shielded from far-field radiation created with the electromagnetic energy. Preferably, a shielding device is used for preventing the far-field radiation to be emitted to the environment. As a particular advantage, unwanted interferences are avoided e.g. in the surrounding measurement equipment or control devices. If the shielding device comprises e.g. a reflecting surface or a resonant cavity, a compact structure of the shielding device and the imaging device is obtained.

Generally, the term "near-field" (or: field of the near-field region or near-field zone) has the meaning as conventionally used in particular in electrodynamics and telecommunication technologies. According to certain definitions, it refers to the electromagnetic field in the neighboring region of the near-field source device where the angular field distribution of the emitted electromagnetic energy is dependent upon the distance from the near-field source device. Typically, it is the part of the radiated field that is within a fraction or a small number of wavelengths of the near-field source device. Furthermore, near-field region could be defined as a non-radiative region of an electromagnetic source where electric and magnetic field vectors are not perpendicular (orthogonal) to each other. This is as opposed to Fraunhofer region where radiation-type field occurs. The near-field might consist of the reactive near field, also known as quasi-static near-field, and the radiating near-field also known as the electromagnetic field in the Fresnel zone or Fresnel region. With a preferred embodiment of the invention, the near-field region of the near-field source device, e. g. one or more antenna elements thereof, extends up to a distance smaller than $2D^2/\lambda$ from it, $\lambda$ being the wavelength of the energy absorbed in the near-field and D being the overall (largest) dimension of the near-field source device, in particular the antenna elements.

However, in many applications, it turns out difficult to define what is the overall (largest) dimension of the near-field source device as it is also difficult to describe which parts of the particular apparatus comprise the source of electromagnetic energy. Furthermore, for efficient emission of electromagnetic energy into the surrounding medium, the source devices (antennas) are usually made with dimensions D on the order or slightly smaller than the wavelengths $\lambda$ emitted by it. Therefore, in order to simplify definitions, according to a particularly preferred embodiment of the invention, the near-field region of the near-field source device is assumed a distance smaller than $\lambda$ from the device, $\lambda$ being the wavelength of the energy absorbed in the near-field region. In cases where a matching medium surrounds the source device, the wavelength should be normalized by an effective refractive index of this particular medium. This definition places objects in close proximity to the emitting element, in the near-field region of the source element; i.e. in a non-radiating region. For example, for a wave with a frequency of interest of 100 MHz, the near-field region in air is at distances less than 3 m and in water is less than 33 cm.

The term "mechanical wave response" generally refers to the generation of mechanical waves in response to the absorption of electromagnetic energy in the object to be investigated, in particular the ROI. The mechanical waves can comprise acoustic waves, pressure waves, shear waves, shock waves, thermal waves or any other kind of acoustic or mechanical disturbance including an interference response, created by interfering mechanical waves, especially when using phased-array source elements. Advantageously, the corresponding acoustic response, pressure response, shear response, shock response or thermal wave response can be detected with available detectors configured for sensing a mechanical oscillation, in particular with at least one of acoustic detectors, optical detectors and interferometric detectors. As a preferred example, the acoustic detector comprises at least one or several piezoelectric detector elements (based on e.g. PZT or PVDF) or capacitive micromachined ultrasound transducers (CMUT). With optical and/or interferometric detectors, oscillations of the object surface caused by mechanical waves can be detected.

With a particularly preferred NRT embodiment, at least one near-field absorbing substance is used for increasing the information content in the image data by enhancing the local contrast and/or by providing additional functional or molecular information in the ROI, e.g. living tissues. The near-field absorbing substance comprises an exogenous agent or probe, which has an increased localized absorption of electromagnetic radiation at the RF frequencies.

These agents could have ferromagnetic or superparamagnetic properties with preferential absorption in the radiofrequency spectrum. Superparamagnetic substances exhibit ferromagnetic resonance in the low MHz frequency range, thus the power absorption by superparamagnetic nanoparticles in the VHF band will be significantly higher as compared to the microwave region utilized in the current thermoacoustic methods. It worth noting that nanoparticles were already successfully employed for improving effectiveness of thermal ablation (hyperthermic) treatments. Examples of some commercially available or existing agents that are also applied in the current MRI studies are monocrystalline iron oxide nanoparticles (also known as Combidex® or MION), cross-linked iron oxide nanoparticles (CLIO) or Gadolinium-based agents. Descriptions can be found in e.g. US patent 4 770 183. Alternatively, a different kind of compounds can be used as near-field absorbing substance, e.g. ceramics as described in US patent 6 662 040. Yet, many other materials that have conducting, semi-conducting, or magnetic loss properties, making them efficient absorbers in the radiofrequency band, can be used instead. Therefore, other radiofrequency absorbing substances may be developed or utilized with the method taught herein. These agents can also be in the form of single molecules, molecule aggregates, other particles, enclosures such as micelles or loaded cells and other agents or agent delivery vehicles. The particles could also be conjugated with antibodies or peptides to enable their binding to specific sites within tissues, thus allowing specific targeting of cellular and sub-cellular moieties for the suggested near-field imaging method. Finally, by increasing the delivered power, selective ablation of nanoparticle binding sites is possible as well. The resulting system may be used for high-resolution non-invasive tomography of biological tissues, in-depth targeted imaging of molecular function and gene expression in-vivo, blood flow characterization, treatment and inflammation monitoring, targeted hyperthermic treatments, and other biomedical applications.

Preferably, the image data obtained with the NRT technique are further processed for reconstructing an image of the ROI. Advantageously, the image may comprise any ROI representation describing one-dimensional, two-dimensional or three-dimensional properties of the ROI. The type of image can be selected in dependency on the particular application of the invention. The inventive system allows a provision of images even with higher dimensions or in complex parameter spaces, e.g. image series taken in dependency on time and/or another physical or chemical condition of the object. Typically, the reconstructed image of the ROI is displayed, whereas usual displaying techniques can be used (e.g. displaying on a screen, printing or the like), and/or subjected to further image processing. With biological or medical applications of the invention, the reconstructed image can be used for a subsequent diagnostic or monitoring step. In addition a hybrid image may be produced by displaying multiple parameters on one image, such as structural and functional or molecular parameters, or by combining near-field tomography images with data or images from another modality.

If according to a further preferred embodiment of the invention the position of the object, the near-field source device and/or the detector device is changed relative to at least one of the other components, the steps of energy input and detection advantageously can be optimized. In particular, a position adjustment can be provided before the first data acquisition or during the data acquisition. Advantageously, the above components can be subjected to at least one of a rotation and a translation so that all geometrical degrees of freedom can be used for improving the imaging quality.

Further embodiments of the invention are related to the use of a matching medium, such as matching fluid or gel. The matching medium can be provided between the near-field source device, e. g. the antenna elements, and the ROI. Accordingly, the coupling efficiency of the electromagnetic energy is improved. Furthermore, the matching medium can be simultaneously used for improving the detection of the mechanical wave response. Alternatively, the near-field electromagnetic energy can be provided without any matching medium. The electromagnetic energy is emitted from the near-field source device, possibly via the gas phase (atmospheric environment) to the object including the ROI. In this case, advantages are obtained in terms of reduced disturbance of the object and facilitated imaging, in particular with medical imaging. Correspondingly the mechanical wave can be detected via the use of a matching medium, or by placement of the detector on the object imaged, or by invasively introducing it into the object or by a non-contact approach.

Another essential advantage of the invention is given by the fact, that there are no restrictions with regard to the object to be investigated. In particular, the object may comprise biological material, like biological tissue or a part thereof. The imaging can be conducted in vivo or *in vitro.* The biological material may originate from animal or human sources or from plant sources. Alternatively, the object may comprise non-biological material. As examples, a work piece, a consumer good or food/drink or a geological structure, e.g. the ground can be investigated for detecting certain parts or conditions of the respective material. Typically, the size of the object or the distance of the ROI below the object's surface can be varied selected in the range of some cm, e. g. 5 cm, up to 50 cm. However, there are no basic limitations imposed by the imaging method, therefore, by changing duration of the emitted pulses and detector characteristics, both the size and the imaging depth can be scaled from below 1 mm to the order of several meters and more.

Biological or medical applications of the invention preferably include imaging or screening of disease or an abnormal condition in animals or humans, such as an inflammatory process in biological tissue, a tumor or cancer in biological tissue, a vascular abnormality or disease in biological tissue, a neurological disease and/or monitoring a treatment of biological tissue. Similarly the method can be used to study normal tissue such as in cases of studying physiology, neurological function, cellular and tissue growth, development, aging etc.

### Brief description of the drawings

Further details and advantages of the invention are described in the following with reference to the attached drawings. The drawings show in:

Figure 1:        a schematic illustration of an embodiment of the inventive imaging device;

Figure 2:        a circuit diagram of a generator device used for the inventive imaging technique; and

Figures 3 to 5:    schematic illustrations of applications of the invention in imaging of biological or non biological objects.

### Description of the preferred embodiments

With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taker with the drawings making apparent to those skilled in the art how the several forms of the invention may be embodied in practice. As used herein, an element or step recited in the singular and proceeded with the word "a" or "an" should be understood as not excluding plural elements or steps, unless such exclusion is explicitly recited. In the description of the figures, like numbers refer to like parts. The drawings are generally not to scale. For clarity, non-essential elements were omitted from some of the drawings. Some optional elements may be drawn in dashed lines.


### 1. Embodiments of the imaging method and device

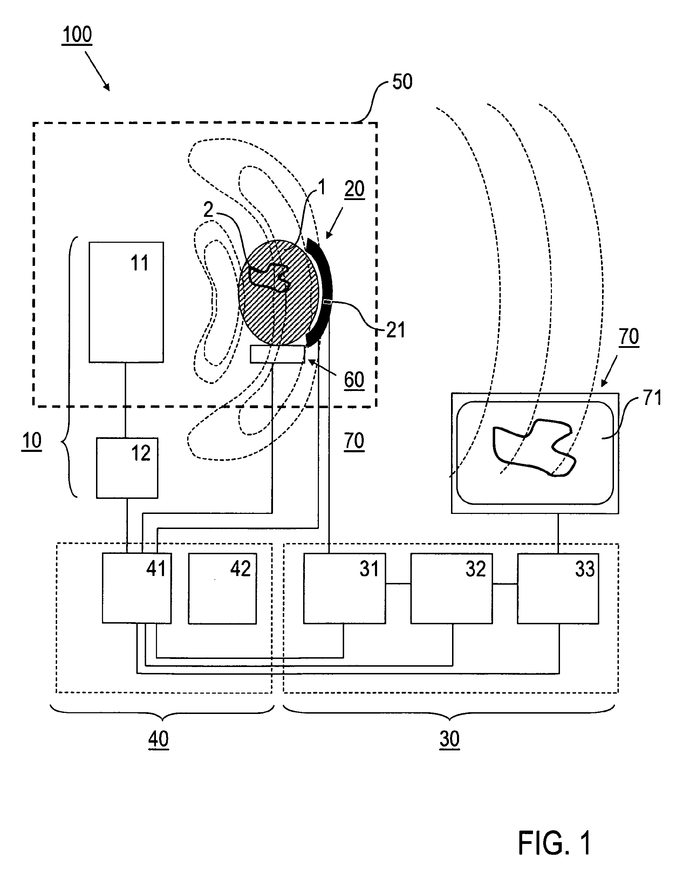

Figure 1 schematically depicts the main building blocks of the imaging device 100 according to a preferred embodiment of the present invention. The imaging device 100 comprises an energy input device 10, a detector device 20, an image processing device 30, a control device 40, a shielding device 50, a carrier device 60 and a display device 70, which are described with further details in the following.

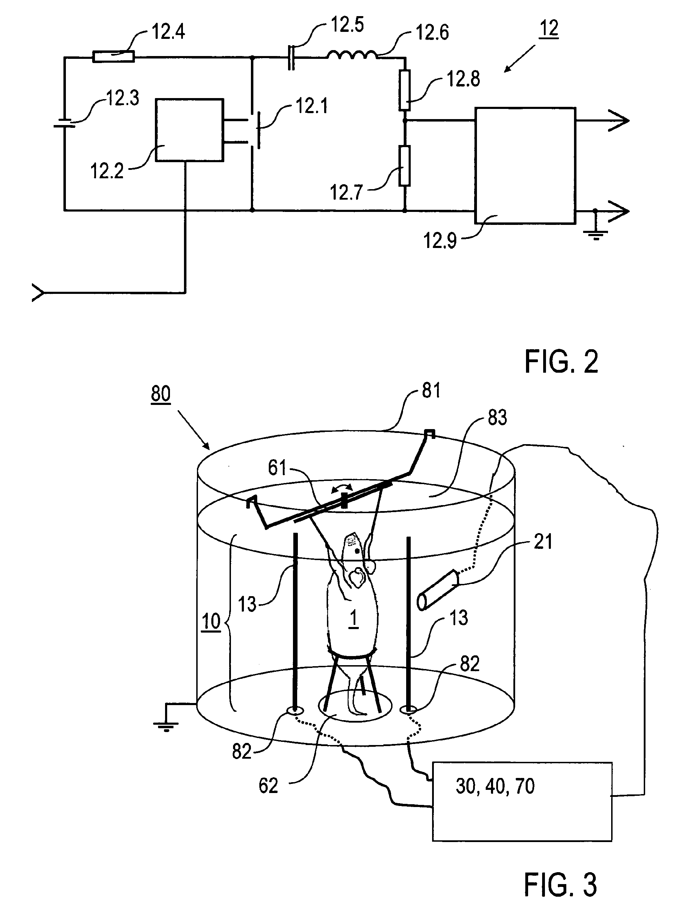

The energy input device 10 includes the near-field source device 11 and a generator device 12. The near-field source device 11 is located in the vicinity of the imaged object 1 including an ROI 2 to be investigated, e. g. within a distance of about 3 cm from the object's surface. The emitting element/s of the near-field source device 11 may consist of one or several of the following: a single or multiple antenna elements, phased array, resonant cavity, waveguide or any other element/s capable of emitting electromagnetic energy into the surrounding or contained medium. In the preferred embodiment, the near-field source device 11 should have sufficiently wideband characteristics albeit can efficiently transmit the broadband impulses fed into it. This is in order to deliver a maximal amount of impulses energy into the object and as evenly as possible. The generator device 12 (further details see Figure 2) is adapted for supplying electrical energy to the near-field source device 11. It is connected either directly or through an appropriate high-voltage radio-frequency cabling and connectors into the energy emitting element/s of the near-field source device 11.

The detector device 20 is adapted for detecting a mechanical wave response generated in the object 1. The detector device 20 comprises e.g. an acoustic detector including at least one piezoelectric detector element 21. Typically, the acoustic detector is structured as it is known from conventional thermoacoustic imaging devices or ultrasound imaging devices. Accordingly, for tomographic applications a plurality of piezoelectric detector elements 21 are arranged at different locations relative to the object 1. Alternatively, when a single detector element 21 is used for the reconstruction, it could be placed on a stepper motor and/or rotation stage (not shown) as a part of tomographic scanning procedure.

The image processing device 30 includes a signal processor circuit 31, a storage circuit 32 and an image reconstruction circuit 33. The control device 40 includes a controller circuit 41, which is adapted for controlling the near-field source device 10, the detector device 20 and the carrier device 60, and a carrier controller 42. Accordingly, the controller circuit 41 is connected with the above components of the image processing device 30. The image processing device 30 and the control device 40 can be provided by separate circuits (as shown) or by a common circuit.

The carrier device 60 is adapted for accommodating the object 1 and for adjusting the position and/or orientation of the object 1 relative to the near-field source device 11 and the detector device 20. To this end, the carrier device 60 includes a drive unit (not shown), which is capable of subjecting the object 1 to rotation and/or translation movements. The carrier device 60 can comprises further stages (not shown) being adapted for adjusting the near-field source device 11 and/or the detector device. In particular for tomographic reconstruction in two or three dimensions, the imaged object 1 and/or detector element/s 21 and/or emitting element/s of the near-field source device 11 could be placed on rotation and/or translation stages, controlled by the carrier controller 42.

The shielding device 50 represents an optional feature of the inventive imaging device 100. As an example, the shielding device 50 comprises a reflecting screen 51, e.g. a grid made of copper. Alternatively, the shielding device 50 may comprise a resonant cavity covering the near-field source device 10, the object 1 and the detector device 20.

As an exemplary illustration, Figure 1 shows the display device 70 being a display screen 71. Alternatively or additionally, a printer or any other device for image formation can be provided as the display device.

According to a preferred embodiment of the inventive method, the generator device 12 is configured for emitting broadband impulses with duration on the order of a single or several nanoseconds. The resulting mechanical response of the object is recorded by the single or multiple detecting element/s 21 of the detector device 20, amplified, digitized and processed by the signal processor circuit 31 and stored on the storage circuit 32. In particular, the signals detected by the ultrasonic detectors elements 21 are amplified and digitized using digital acquisition hardware. The stored data is further processed by the image reconstruction circuit 32, which conducts a reconstruction algorithm, and the resulting image is presented on the display screen 71. Alternatively, a real-time reconstruction process using a beam forming hardware or software algorithm is applied directly on the data obtained from the detector elements 21. The main elements of the system are controlled via the control device 40, which comprises e. g. a PC.

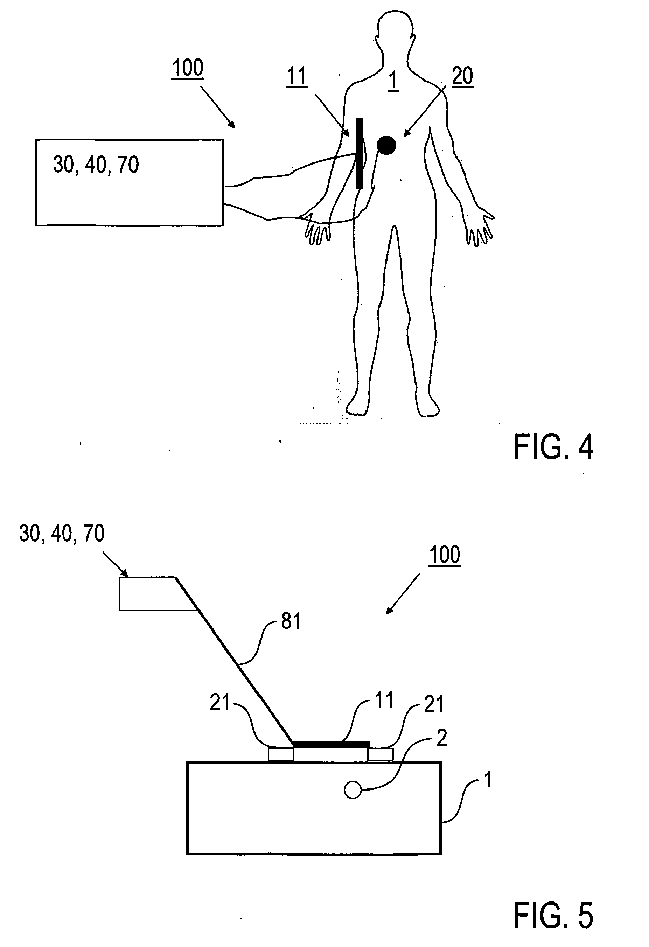

The near-field source device 11, the object 1 and the detector device 20 could be enclosed in a shielded container filled with water or matching fluid (see Figure 3). It'll also prevent from strong electromagnetic disturbances to propagate outside the setup. Instead, a non-contact scheme could be applied with the emitting element (antenna) located in the open air in the vicinity of the imaged area and a phased array with beam forming algorithm used for signal detection (see Figure 4). In this embodiment, there is no need in matching fluid and the images can be obtained in real-time using delay-and-sum or other beam forming algorithms, as is usually done in ultrasonic imaging.

It should be pointed out that the optimal dimensions of the radiating antenna operating in air will be generally significantly larger than those for water due to the longer wavelength of the emitted frequency content.

### 2. Generator device

The pulsing circuit of the generator device 12, whose schematics is depicted in Figure 2, is designed to produce ultrashort (single or several nanosecond FWHM) duration broadband impulses having about 100 Hz repetition frequency with peak voltage of several tens of Kilovolts sufficient for generating about a Watt of average output power. It is designed to use a relatively inexpensive triggered spark gap 12.1 instead of a costly hydrogen thyratron commonly used in pulsing applications.

The main pulsing circuit consists of the triggered spark gap 12.1 with a trigger pulse generator 12.2, an adjustable negative high voltage power supply 12.3, a charging resistor 12.4, a high voltage capacitor 12.5, a risetime limiting inductance 12.6, a back termination resistor 12.7, an optional series resistance 12.8 and a transmission line step-up transformer 12.9.

The power supply voltage is adjustable with a front panel control. The voltage is monitored by a front panel digital meter. The capacitor charges through the charging resistor 12.4 and the back termination resistor 12.7 and the load resistance if the load has a dc path to ground. When a trigger pulse is received on the trigger input, the spark gap 12.1 is fired which dumps the capacitor 12.5 into the output circuit. The capacitor 12.5 is a specially constructed low inductance high voltage capacitor. The pulse width is determined by the capacitance 12.5 and the parallel combination of the load resistor and the output coax characteristic impedance. The value can be changed by increasing or decreasing the high voltage capacitor 12.5. The back termination resistor 12.7 absorbs reflections from the load to insure that only a single pulse of the desired amplitude is delivered to the load, independent of the load impedance. The expected lifetime of the triggered spark gap 12.1 can comprise many millions of pulses due to the low charge per shot and the low average current.

### 3. Image formation

Generally, image reconstruction on the basis of the image data obtained with the invention is provided with algorithms or procedures as known from thermoacoustics. If the image reconstruction requires information on the structure of the near-field, the latter can be analytically, iteratively or numerically calculated, or measured with a phantom sample.

A broadband acoustic radiation is induced in tissue following the instantaneous temperature elevation caused by absorption of short pulses of radio-frequency energy in matter. The magnitude of the induced acoustic waves is proportional to the local energy density, electrical conductivity, and thermoelastic properties of the object 1. Their spectrum, in turn, is mainly dependent upon the spatial frequency of energy deposition variations and duration of the emitted pulses. For pulse durations in the ns range, a biologically relevant thermoacoustic spectrum will be of ultrawideband nature with useful information contained between several tens of kHz and several tens of MHz, depending on size of the imaged object

1. Preserving the correct shape of the detected response is important for the correct quantification of the resulting images. Since it may be difficult to effectively implement such a broadband detection, the preferred way to restore the initial tissue response is to deconvolve the recorded from the frequency response of the detector. Alternatively, ultrawideband detection approaches may be used, such as optical interferometric approaches based on detection of mechanical oscillations in optically resonant elements, e.g. Fabry-Perot films, ring resonators, or ethalons. Generally, under conditions of heat confinement, i.e. when the electromagnetic energy pulse is short enough so that the thermal diffusion is insignificant during the pulse, the spatio-temporal dependence between thermoacoustically-induced pressure $p(\vec{r},t)$, absorbed energy density $U((\vec{r},t)$ (in $J/m^3$) and local temperature elevation $T(\vec{r},t)$ can be expressed as

$$\nabla^2 p(\vec{r},t) - \frac{1}{v_s^2}\frac{\partial^2 p(\vec{r},t)}{\partial t^2} = -\rho_m \beta \frac{\partial^2 T(\vec{r},t)}{\partial t^2} = -\frac{\beta}{C}\frac{\partial U(\vec{r},t)}{\partial t} \qquad (1)$$

where $v_s$, $\rho_m$, $\beta$, and $C$ are the corresponding speed of sound, mass density, isobaric volume expansion, and specific heat of the medium, all are in general spatially and frequency dependent.

In practice, the thermal confinement conditions are fulfilled for excitation pulse durations less then 1 $\mu$s. When a point ultrasonic detector is placed in the position $\vec{r}$ it will sense an integrated pressure wave, which is the solution of (1), namely,

$$p(\vec{r}',t) = -\frac{\beta}{4\pi C}\int_V \frac{\partial U(\vec{r},t')}{\partial t'}\frac{d^3\vec{r}}{|\vec{r}-\vec{r}'|}\bigg|_{t'=tv_c} \qquad (2)$$

The goal of the suggested near-field imaging method is to reconstruct the e. g. three-dimensional energy absorption distribution from a set of measured ultrasonic pressures $p(\vec{r},t)$.

In its simple form this can be done by backprojecting the detected signals into the virtual imaged volume or by various Radon transformations. However, the specific inversion scheme will differ in each case depending on particular geometrical and physical characteristics and spatial distribution of the detection elements used. For example, in case a phased-array of detector is used, the images can be formed in the real-time by using the simple ultrasound beam forming algorithms.

The basic result of the inversion of Equ. (2) can be presented in a form of image/s representing local near-field power deposition in tissue. Introducing near-field absorbing agents into specific target regions in the object will increase the local power deposition in those regions, which in turn will enhance the anatomical, functional or molecular contrast and visualization capabilities in the presented images.

4. Applications

There is a wealth of applications for the invented method. While not limited only to the biomedical field, the application of the technique to biomedical imaging is an important direction.

4.1 Biomedical imaging

Figure 3 illustrates a preferred application of the inventive technique in biomedical imaging of small animals, like mice or other rodents. A container device 80 is provided, which comprises a tank 81 and holding elements 82, which are adapted for positioning components of the imaging device 100. The tank 81 contains a matching fluid, e.g. water or oil. The object to be investigated (living mouse 1) is positioned in the tank 81 using the carrier device with a hang-up portion 61 and a carrier portion 62. The hang-up portion 61 is adapted for accommodating a first part of the object, e. g. the legs of the mouse. The hang-up portion 61 is rotatable relative to the near-field source device 10 and the detector device 20 (see double-arrow). The carrier portion 62 includes a rotatable platform adapted for accommodating a second opposite part of the object.

The near-field source device 11 comprises two rod-shaped antenna elements 13 (monopole elements). The antenna elements 13 are arranged with a parallel relationship relative to the carrier device along the longitudinal distance between the hang-up and carrier portions 61, 62. The detector device 20 comprises an array of acoustic detectors 21. The detector device 20 is arranged in the neighbourhood of the near-field source device 10 and the mouse 1. Advantageously, there are no particular restrictions with regard to the position of the detector device 20. For implementing the above image reconstruction, it is only necessary to have an information on the location of the detector elements 21 relative to the object (mouse 1).

The near-field source device 11, in particular the antenna elements 13, and the detector device 20 are connected with the components 30, 40 and 70 (Figure 1). These components are arranged outside the container device 80.

With alternative embodiments of the invention, the carrier device can be provided with fixed hang-up and carrier portions, multiple antenna elements and/or detector elements can be circumferentially arranged on all sides of the object, and/or the antenna and/or detector element can be rotatable around the object.

The tank 81 fulfils the double-function of accommodating the matching fluid 83 and shielding the environment of far-field radiation emitted from the antenna element 13. To this end, the tank 81 consists of or is coated with a reflecting material, like e.g. metal. Furthermore, the reflecting material 81 is connected with ground.

The embodiment schematically illustrated in Figure 3 is not restricted to the investigation of small animals. Alternatively, other biological objects can be imaged, e.g. human beings or larger animals or parts thereof. As an example, tank 81 can be adapted for accommodating a part of a human patient instead of the mouse 1.

4.2 Clinical imaging

Areas of preferred clinical applications include imaging of cardiovascular disease, cancer, inflammation and neurodegenerative disease, to name a few examples. Imaging of natural states such as growth and aging are also contemplated. As a particular advantage, the inventive near-field imaging can be conducted without using a matching fluid between the near-field source device and the object to be investigated, thus essentially facilitating the clinical applications.

Figure 4 schematically illustrates an embodiment whereas the invention is used for imaging a human being or a part thereof, e. g. an organ. The near-field source device 11, which comprises e.g. a single rod-shaped antenna element is arranged outside the body of the human patient 1. The distance between the antenna element and the outer surface of the human patient 1 greatly depends on the size of region-of-interest and spectrum emitted by the generator and may be selected in the range of 5 mm to 50 cm for most applications. The detector device 22, which comprises an array of detector elements 21 is arranged on the outer surface of the human patient 1 as well. For improving the acoustic detection, a matching gel can be provided between the outer skin of the human patient 1 and the detector device 20. Both, the near-field source device 11 and the detector device 20 are connected with the components 30, 40 and 70 (Figure 1).

In a preferred embodiment an agent is injected intravenously or locally to the patient 1, and targets areas or processes of interest. The near field emitting source (antenna element 13) is brought in the proximity of the tissue so that near-field energy is optimally coupled into it. Subsequently, the mechanical wave detector device 20, such as an acoustic detector or array of acoustic detectors 21 detects acoustic waves generated within the tissue. The collected data is processed and presented in the form of two- or three-dimensional image on the monitor. Ideally, the apparatus is capable of real-time (30 or more frames per second) visualization of the imaged cross-section volume and specific bio-markers contained in it.

An application example includes the administration of iron-oxide base nanoparticles (size e. g. on the order of 10 - 100 nm) that are preferentially uptaken by macrophages. Image of their absorption yields areas of increased inflammation as in the case of image atherosclerotic plaque, in the carotids or other vessels. Similarly targeted absorbing particles can show information on targeted molecules such as peptides, receptors etc.

Another application example includes imaging of the effect of treatment, via drugs, radiation or chemotherapy, by similarly administrating absorbing particles in the body and monitoring their relative update or targeting over time.

In other embodiments, the same detection can be achieved by portable devices, or endoscopic devices inserted into body cavities or through invasive procedures by operatively inserting the device into the tissue.

4.3 Further applications

An example of investigating non-biological samples is schematically illustrated in Figure 5. The imaging device 100 is provided as a hand-held, portable device including the near-field source device 11 and the detector device 20 at a free end of a support rod, whereas the components 30, 40 and 70 are arranged at the opposite and thereof. Components 30, 40 and 70 can be included in a box with a handle. The object 1 comprises e.g. food or a container, in which a certain component (ROI 2) is to be investigated. Other non-biological samples like ground or other geological structure can be investigated in an analogue way.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention.

**Claims**

**1.** A method of near-field imaging of a region of interest, comprising the steps of:

- emitting an input of near-field electromagnetic energy from a near-field source device into the region of interest,
- detecting a mechanical wave response generated in the region of interest in response to the near-field electromagnetic energy input using a detector device, and
- providing image data representing an image of the region of interest on the basis of the mechanical wave response.

2.  The method of claim 1, wherein

- the region of interest is placed within the near-field region of the near-field source device at a distance smaller than λ from the near-field source device, λ being a wavelength of the electromagnetic energy absorbed in the near-field.

3.  The method of claim 1, wherein

- the near-field electromagnetic energy is emitted as a pulse modulated signal.

4.  The method of claim 3, comprising the step of

- performing at least one of amplitude, frequency, phase or digital modulation of the pulse modulated signal.

5.  The method of claim 1, wherein

- the near-field energy is emitted as a modulated intensity signal.

6.  The method of claim 5, comprising the step of

- performing at least one of amplitude, frequency, phase or digital modulation of the intensity modulated signal.

7.  The method of claim 1, comprising the step of

- preventing emission of far-field radiation into a surrounding region.

8.  The method of claim 1, wherein

- the mechanical wave response comprises at least one of an acoustic response, a pressure response, a shear wave response, a shock wave response, an interference response or thermal waves response.

9.  The method of claim 1, wherein

- the mechanical wave response is detected by the detector device including an acoustic detector or an optical or interferometric detector.

10. The method of claim 9, wherein

- the detector device comprises at least one detector element or a plurality of detector elements arranged in an array format.

11. The method of claim 1, comprising the step of

- reconstructing the image of the region of interest from the image data, wherein
- the image describes at least one of one-dimensional (depth profiling), two-dimensional (surface or cross-sectional) and three-dimensional (volumetric) properties of the region of interest.

12. The method of claim 11, comprising the step of

- displaying the image of the region of interest.

13. The method of claim 1, comprising the step of

- adjusting a relative position or orientation of at least one of the object, the near-field source device and the detector device relative to each other before or during data acquisition.

**14.** The method of claim 13, wherein

- the adjusting step comprises at least one of a rotation and a translation of at least one of the object, the near-field source device and the detector device.

**15.** The method of claim 1, wherein

- the region of interest comprises or contains biological tissue, or
- the region of interest comprises at least one of a biological plant or a part thereof, a work piece, a consumer good or a geological structure.

**16.** The method of claim 1, comprising the step of

- depositing a near-field electromagnetic energy absorbing substance in the region of interest.

**17.** The method of claim 16, wherein

- the near-field absorbing substance is a material having electromagnetic absorption in the radiofrequency band.

**18.** The method of claim 17, wherein

- the near-field absorbing substance is a material exhibiting paramagnetic, superparamagnetic, ferromagnetic, conducting or semi-conducting properties.

**19.** The method of claim 16, wherein

- the near-field absorbing substance is capable of specifically binding with at least one of proteins, receptors, enzymes, a physiological structure, a cellular structure and a sub-cellular structure.

**20.** The method of claim 16, wherein

- the near-field absorbing substance is capable of specifically responding to at least one of physiological, cellular and sub-cellular activity.

**21.** The method of claim 16, where the substance is systemically administered in tissue.

**22.** The method of claim 1, comprising at least one of the steps of

- imaging of pathological tissue, a disease or an abnormal condition in animals or humans,
- detecting an inflammatory process in biological tissue,
- detecting a tumor or cancer in biological tissue,
- detecting a carotid abnormality in biological tissue,
- monitoring a treatment of biological tissue.
- performing molecular imaging.

**23.** An imaging device adapted for near-field imaging a region of interest in an object, comprising:

- a near-field source device being arranged for emitting an input of near-field electromagnetic energy into the region of interest,
- a detector device being arranged for detecting a mechanical wave response generated in the region of interest in response to the near-field electromagnetic energy input, and
- an image processing device being arranged for providing image data representing an image of the region of interest on the basis of the mechanical wave response.

**24.** The imaging device of claim 23, wherein

- the near-field source device is connected with a generator source device adapted for generating a pulse modulated radio-frequency signal or a modulated intensity signal providing the input of near-field electromagnetic energy.

**25.** The imaging device of claim 23, wherein

- the generator source device is adapted for generating electromagnetic impulses with full-width-half-maximum duration between 10 ps and 10 $\mu$s or electromagnetic bursts (pulses) with duration between 1 ns and 100 $\mu$s.

**26.** The imaging device of claim 25, wherein

- the generator source device includes at least one of a triggered spark gap, avalanche diode, ionization diode, vacuum tube, e.g. klystron, gas-filled tube, e.g. thyratron or a magnetron.

**27.** The imaging device of claim 23, comprising

- a shielding device preventing emission of far-field radiation to a surrounding region of the object.

**28.** The imaging device of claim 27, wherein

- the shielding device comprises a reflecting surface or a resonant cavity.

**29.** The imaging device of claim 23, comprising

- a carrier device adapted for accommodating the object in positional relationship relative to the near-field source device and the detector device.

**30.** The imaging device of claim 23, wherein

- at least one of the carrier device and the near-field source device is adapted for positioning the object in a near-field region of the near-field source device at a distance smaller than $\lambda$ from it, $\lambda$ being a wavelength of the energy absorbed in the near-field.

**31.** The imaging device of claim 23, wherein

- at least one of the carrier device and the near-field source device is adapted for positioning the object in a near-field region of the near-field source device at a distance smaller than $2D^2/\lambda$ from it, $\lambda$ being a wavelength of the energy absorbed in the near-field and D being the overall (largest) dimension of the near-field source device.

**32.** The imaging device of claim 23, wherein

- at least one of the carrier device and the near-field source device is adapted for positioning the object in a near-field region of the near-field source device, wherein the near-field region is a non-radiative region of the near-field source device.

**33.** The imaging device of claim 23, wherein

- the near-field source device is adapted for creating far-field radiation, and
- at least one of the carrier device and the near-field source device is adapted for positioning the object in the near field of the near-field source device.

**34.** The imaging device of claim 23, wherein

- the detector device is adapted for detecting at least one of an acoustic response, a pressure response, a shear response, a shock response, an interference response or thermal waves response.

**35.** The imaging device of claim 34, wherein

- the detector device includes an acoustic detector or an optical or interferometric device.

**36.** The imaging device of claim 35, wherein

- the detector device comprises at least one detector element or a plurality of detector elements arranged in an array format

**37.** The imaging device of claim 23, wherein

- the image processing device is adapted for reconstructing the image describing at least one of one-dimensional (depth profiling), two-dimensional (surface or cross-sectional) and three-dimensional (volumetric) properties of the region of interest.

**38.** The imaging device of claim 23, wherein

- the near-field source device and the detector device provide a stationary, portable or handheld system.

**39.** The imaging device of claim 23, wherein

- the near-field source device and/or the detector device are used non-invasively.

**40.** The imaging device of claim 23, wherein

- the near-field source device and/or the detector device are used invasively, e. g. interstitially or intravenously or in body cavities, e.g. nasal, esophagus, lungs.

FIG. 1

FIG. 2

FIG. 3

FIG. 4

FIG. 5

**European Patent Office**

## EUROPEAN SEARCH REPORT

Application Number

EP 08 00 6528

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 459 392 A (HITACHI LTD [JP]) 4 December 1991 (1991-12-04) | 1,2,5,6, 8,9, 11-15, 22,23, 26, 29-32, 35,37-39 | INV. A61B5/05 A61B8/00 G01N21/17 |
| Y | * page 2, line 1 - line 4 *  * page 7, line 7 - page 9, line 22 * * page 16, line 12 - line 46 * * claims 1-14 * * figure 1 * ----- | 7,27,28, 40 | |
| Y | EP 1 333 703 A (LG ELECTRONICS INC [KR]) 6 August 2003 (2003-08-06) * abstract * ----- | 7,27,28 | |
| X | JP 2007 307007 A (HAMAMATSU KAGAKU GIJUTSU KENKY) 29 November 2007 (2007-11-29) | 1,23 | |
| Y | * abstract * * paragraph [0001] - paragraph [0002] * ----- | 40 | TECHNICAL FIELDS SEARCHED (IPC) A61B G01N H05B |
| X | US 6 567 688 B1 (WANG LIHONG [US]) 20 May 2003 (2003-05-20)  * column 2, line 8 - line 52 * * column 4, line 20 - column 5, line 53 * * column 14, line 18 - line 29 * * figure 2 * ----- | 1-4, 8-15,24, 25,30, 32-37 | |

The present search report has been drawn up for all claims

| Place of search | Date of completion of the search | Examiner |
|---|---|---|
| Munich | 19 September 2008 | Montes, Pau |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 08 00 6528

This annex lists the patent family members relating to the patent documents cited in the above-mentioned European search report.
The members are as contained in the European Patent Office EDP file on
The European Patent Office is in no way liable for these particulars which are merely given for the purpose of information.

19-09-2008

| Patent document cited in search report | | Publication date | Patent family member(s) | | Publication date |
|---|---|---|---|---|---|
| EP 0459392 | A | 04-12-1991 | DE | 69131528 D1 | 23-09-1999 |
| | | | DE | 69131528 T2 | 04-05-2000 |
| | | | US | 5214282 A | 25-05-1993 |
| EP 1333703 | A | 06-08-2003 | CN | 1435599 A | 13-08-2003 |
| | | | JP | 2003223985 A | 08-08-2003 |
| | | | KR | 20030065728 A | 09-08-2003 |
| | | | US | 2003141298 A1 | 31-07-2003 |
| JP 2007307007 | A | 29-11-2007 | NONE | | |
| US 6567688 | B1 | 20-05-2003 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4255971 A, A. Rosencwaig
- US 6567688 B
- US 6216025 B
- US 4343993 A
- US 7005653 B
- US 4770183 A
- US 6662040 B

**Non-patent literature cited in the description**

- **Ash, E.A. et al.** Super-Resolution Aperture Scanning Microscope. *Nature,* June 1972, vol. 237, 510-512